# EUROPEAN PATENT APPLICATION

(11) **EP 2 446 891 A1**
(43) Date of publication of application: **02.05.2012**
(21) Application number: 10306184.2
(22) Date of filing: 28.10.2010
(51) Int. Cl.: A61K 31/727

(54) **Semuloparin for use as an antithrombotic treatment in major abdominal surgery with improved safety in terms of clinically relevant bleedings and major bleedings**

(71) Applicant: Aventis Pharma S.A., 92160 Antony (FR)
(72) Inventor: The designation of the inventor has not yet been filed
(74) Representative: Romanowski, Caroline

(57) **Abstract**

The invention relates to the use of semuloparin or a pharmaceutically acceptable salt thereof as an antithrombotic treatment in patients undergoing major abdominal surgery, wherein said use involves an improved safety in terms of clinically relevant bleedings and of major bleedings compared to a standard antithrombotic treatment.

## Description

The invention relates to the use of semuloparin or a pharmaceutically acceptable salt thereof as an antithrombotic treatment in patients undergoing major abdominal surgery, wherein said use involves an improved safety, in terms of clinically relevant bleedings and of major bleedings, compared to a standard antithrombotic treatment.

Semuloparin, or AVE5026 (sanofi-aventis laboratory code) belongs to a new generation of hemisynthetic heparins. It is a new ultra-low molecular weight heparin, with an average molecular weight of 2000-3000 Daltons and a novel antithrombotic profile resulting from high anti-Factor Xa activity (- 160 U/mg) and residual anti-Factor IIa activity (- 2 U/mg), as described in Journal of Thrombosis and Haemostasis, 2009, vol. 7, 1143-1151. It is obtained by selective and controlled depolymerization of heparin, as described in the patent application WO 2004/033503 and in J. Thromb Haemost. (ibid). Semuloparin, in the form of its sodium salt, is in clinical development for venous thromboembolism prevention.

At the present time, low-molecular-weight heparins (LMWHs), the synthetic pentasaccharide fondaparinux or dose-adjusted anti-vitamin K are the primary treatments for the prevention of venous thromboembolic diseases.

Patients undergoing general surgery are at substantial risk of postoperative venous thromboembolism (hereafter "VTE"). In addition, many hospitalized patients have additional risk factors for VTE. In order to avoid postoperative venous thromboembolic complication, guidelines in thrombosis (ACCP guidelines) recommend the use of antithrombotic drugs for certain categories of surgical patients. Amongst these drugs, the LMWH enoxaparin is the pharmacological VTE prevention agent with the highest clinical documentation in surgical populations and with the largest clinical use in this setting. Enoxaparin has an average molecular weight of 3800-5000 Daltons, an anti-Factor Xa activity comprised between 90 and 125 IU/mg and an anti-Factor IIa activity of 20-35 IU/mg.

These therapies are effective, but the antithrombotic properties of such drugs are accompanied by a risk of haemorrhage.

The Applicant has now demonstrated that a product outside of the LMWH class, namely the ultra-low molecular weight heparin (ULMWH) semuloparin, displays an advantageous safety profile in terms of bleedings when used in major abdominal surgery.

Therefore, the subject-matter of the invention is semuloparin for use as an antithrombotic treatment in patients undergoing major abdominal surgery, wherein said use involves an improved safety in terms of clinically relevant bleedings, including major bleedings, compared to a standard antithrombotic treatment.

The term semuloparin, in the framework of the instant invention, encompasses any pharmaceutically acceptable salt thereof, in particular its sodium salt. The term "semuloparin" shall therefore be understood as "semuloparin or any pharmaceutically acceptable salt thereof" throughout the instant specification.

According to the instant invention, semuloparin involves a decrease in the incidence of clinically relevant bleedings (including major bleedings) compared to the incidence of clinically relevant bleedings and of major bleedings occurring with a standard antithrombotic treatment.

According to the instant invention, the terms below have the following meanings:
- "treatment" refers to the administration of a therapy to an individual who is considered as being at risk for a thromboembolic pathology, in particular venous thromboembolism (VTE), such as deep vein thrombosis, which may lead to pulmonary embolism. It shall therefore be understood that the term "treatment" as used in the instant invention refers to a prophylactic treatment of venous thromboembolism;
- "patient" refers to a subject being at risk of VTE and for whom prophylaxis of venous thromboembolic events is indicated;
- "major abdominal surgery" refers to open surgery in the peritoneal and/or the retroperitoneal space and/or the pelvis minor for indications other than liver (including hepatobiliary system), uterus, or prostate related, said surgery being performed under general anesthesia, and lasting more than 45 minutes.

In an embodiment of the invention, the patients are undergoing abdominal surgery of the colon, rectum, stomach, small bowel, pancreas, kidneys, ovaries, liver, bladder, or muscle or soft tissues.

In the instant invention, the term "clinically relevant bleedings" designates bleeding events adjudicated by a Central Independent Adjudication Committee (CIAC) as:
- major bleedings, or
- clinically relevant non-major bleedings.

A major bleeding designates a bleeding associated with at least one of the following:
- Fatal bleeding (major contributor to death);
- Symptomatic bleeding in a critical area or organ, such as intracranial, intraspinal, intraocular, retroperitoneal, intraarticular or pericardial, or intramuscular leading to a compartment syndrome;
- Bleeding causing a post-operative fall of hemoglobin level of 2 g/dL (1.24 mmol/L) or more compared to the first post-operative hemoglobin value or the most recent pre-bleeding hemoglobin value, or leading to a post-operative transfusion of two or more units of whole blood or red cells;
- Bleeding leading to an invasive diagnostic or therapeutic intervention (e.g. re-operation, needle aspiration at surgical site, gastroscopy, colonoscopy, ...);
- Circulatory decompensation (related to overt bleeding leading to a systolic blood pressure [SBP] of < 90mmHg or requiring massive fluids infusion or vasopressor support to maintain SBP ≥ 90 mmHg).

A clinically relevant non-major bleeding is defined as:
- Skin hematoma, excluding wound hematoma, requiring surgical or medical intervention. Wound hematoma requiring intervention (e.g. needle aspiration) is adjudicated as a major bleeding according to the definition above (bleeding leading to an invasive diagnostic or therapeutic intervention - e.g. re-operation, needle aspiration at surgical site, gastroscopy, colonoscopy, ...). In addition, an unusual wound hematoma that does not require surgical or medical intervention maybe adjudicated as a clinically relevant non-major bleeding;
- Epistaxis requiring surgical or medical treatment (i.e., packing);
- Macroscopic hematuria not mainly caused by instrumentation (i.e., difficult catheter insertion);
- Unscheduled contact/attention (of Health Care Professional) due to an overt bleeding event but not fulfilling the criteria of a major bleeding (i.e., prolonged bleeding after venopuncture, ...).

In an embodiment of the instant invention, the standard antithrombotic treatment consists in the LMWH known under the INN (International Nonproprietary Name) enoxaparin. Enoxaparin is marketed under the tradenames Lovenox^{®} or Clexane^{®}.

According to the instant invention, semuloparin is administered at a 20 mg daily dose to patients with normal renal function or to patients with mild or moderate renal impairment. For patients with severe renal impairment, semuloparin is administered at a 10 mg daily dose.

According to the instant invention, the renal function of the patients is defined according to estimated creatinine clearance (CLcr) values calculated using the well-known Cockroft-Gault formula, and is classified according to the following characteristics :
- normal renal function: CLcr > 80 mL/min;
- mild renal impairment: 50 ≤ CLcr ≤ 80 mL/min;
- moderate renal impairment: 30 ≤ CLcr < 50 mL/min;
- severe renal impairment: CLcr < 30 mL/min.

The treatment with semuloparin is administered once daily. Said treatment is started post-operatively and is administered for 7 to 10 days.

In the instant invention, the improved safety of semuloparin (i.e., the decrease in the incidence of clinically relevant bleedings and in the incidence of major bleedings) is clinically proven by a phase III clinical trial. It shall be understood that a "phase III clinical trial" refers to a multinational, multicenter, randomized, double-blinded study involving a large patients group, as defined by the Health authorities and the regulatory laws and guidelines, aiming at being the definitive assessment of how effective and safe the drug is in comparison with current standard treatment.

As used herein, the wording "semuloparin for ..." shall be understood as being equivalent to the wording "use of semuloparin for ..." or "use of semuloparin for the preparation of a medicament for use in ...".

The invention therefore also relates to the use of semuloparin or a pharmaceutically acceptable salt thereof for the manufacture of a medicament useful as an antithrombotic treatment in patients undergoing major abdominal surgery, wherein said use involves a decrease in the incidence of clinically relevant bleedings, therefore an improved safety, compared to a standard antithrombotic treatment. All of the embodiments as described above also apply to said use. In particular, said use is clinically proven by a phase III clinical trial.

The invention also relates to an article of manufacture comprising:
- a packaging material,
- a compound chosen from semuloparin or a pharmaceutically acceptable salt thereof, and
- a label or package insert contained within said packaging material indicating that said compound is safe as an antithrombotic treatment in patients undergoing major abdominal surgery.

Having now described the present invention, the same will be more clearly understood by reference to the following examples of the invention, which are included herewith for purposes of illustration only and are not intended to be limiting of the invention.

The following abbreviations shall be used:
BMI: Body Mass Index
CIAC: Central Independent Adjudication Committee
CLcr: creatinine clearance
DVT: deep vein thrombosis
IP: investigational product
PE: pulmonary embolism
UFH: unfractionated heparin
LMWH: low molecular weight heparin
OR: Odds Ratio
q.d.: *quaque die* (once daily)
s.c.: subcutaneously
SRI: severe renal impairment
VTE: venous thromboembolism
95% mid-p CI: 95% mid-p Confidence Interval

### Example 1): Preparation of semuloparin

Semuloparin, in the form of a sodium salt, is obtained by a chemoselective depolymerization of heparin, activated through its benzyl ester derivative, by the phosphazene base 2-tert-butylimino-2-diethylamino-1,3-dimethylperhydro-1,2,3-diazaphosphorine (BEMP). The hemisynthetic pathway, allowing to recover semuloparin in the form of a sodium salt, is described in the patent application WO 2004/033503 and in Journal of Thrombosis and Haemostasis, 2009, vol. 7, 1143-1151. This procedure yields semuloparin with an average molecular weight around 2400 Da, an anti-Factor Xa activity of - 160 U/mg and a residual anti-Factor IIa activity (~ 2 U/mg).

### Example 2): The SAVE-ABDO study.

A Multinational, Multicenter, Randomized, Double Blind Study comparing the Efficacy and Safety of AVE5026 with enoxaparin for the Prevention of Venous Thromboembolism in Patients Undergoing Major Abdominal Surgery.

### 1) Study objectives

The primary objective of the study was to compare the efficacy of once daily (q.d.) subcutaneous (s.c.) injections of 20 mg AVE5026 (10 mg for patients with SRI) with q.d. s.c. injections of 40 mg enoxaparin (20 mg in SRI patients) administered up to Day 7 to 10 for the prevention of venous thromboembolic events in patients undergoing major abdominal surgery. The secondary objectives of this study were to evaluate the safety of AVE5026 in patients undergoing major abdominal surgery and to document AVE5026 exposures in this population.

### 2) Study design

Patient's eligibility was determined during the screening period (within the 14 days prior to surgery) and was reviewed before randomization (day before surgery or day of surgery).

Randomized treatment was allocated to eligible patients, taking into account the geographical region of the patient, the type of surgery (cancer / non-cancer) and the estimated CLcr at baseline (< or ≥ 30 mL/min). Randomization occurred a close as possible before the first IP injection (according to the local enoxaparin labeling, but in any case prior to surgery).

During the study treatment period, signs and symptoms of VTE, bleedings and adverse events, concomitant medications and compliance were assessed. An end of treatment visit was performed the day of last IP injection or at Day 10, whichever came first. A mandatory bilateral venography of the lower limbs was performed at Day 7 to 11 (within one calendar day of the last IP injection) for detection of asymptomatic DVT. A follow-up visit was scheduled at Day 35 to 42.

Maximum duration of study participation was therefore 42 days, including a treatment period up to Day 7-10 and a follow-up period with a visit at Day 35-42 after randomization.

### 3) Patients

A total of 4413 patients were randomized in the study.

### 3-1: Inclusion criteria

Patients meeting the following criteria were suitable for enrolment in the study:
- Patients undergoing major surgery in the peritoneal and/or the retroperitoneal space and/or the pelvis minor (but not limited to the pelvis minor) for indications other than limited disease of the liver (including hepatobiliary system), the uterus, or the prostate; and
- Signed written informed consent.

«Major surgery »was defined as open surgery, under general anesthesia, and lasting more than 45 minutes.

Patients aged less than 60 years must have had one of the following additional risk factors:
- History of VTE,
- Obesity (BMI ≥30 kg/m²),
- Chronic Heart Failure,
- Chronic Respiratory Failure,
- Inflammatory Bowel Disease,
- Cancer surgery.

Definition of the qualifying conditions above for the inclusion in the study:
- Chronic Heart Failure is defined as systolic dysfunction leading to low cardiac output. Chronic heart failure functional classes are be defined according to the New York Heart Association Functional Classes:
   - Class III: patients with cardiac disease resulting in marked limited physical activity. These patients are comfortable at rest. Less than ordinary activity causes fatigue, palpitation, dyspnea, or angina.
   - Class IV: patients with cardiac disease resulting in inability to carry on physical activity without discomfort.

Symptoms of cardiac insufficiency or of the anginal syndrome may be present even at rest. If any physical activity is undertaken, discomfort is increased.
- Respiratory failure can be manifest either as hypoxemia, hypercarbia, or a combination of both types of gas exchange abnormalities. Patients with chronic respiratory failure may have any of the following underlying pulmonary diseases:
   - Obstructive: chronic obstructive pulmonary disease (COPD) (chronic bronchitis, emphysema).
   - Restrictive parenchymal (alveolar and interstitial lung disease) (like sarcoidosis, idiopathic pulmonary fibrosis, pneumoconiosis, connective tissue disorders, drug or radiation induced lung disease).
   - Restrictive extraparenchymal lung disease due to neuromuscular disorders (like diaphragmatic paralysis, myasthenia gravis, Guillain Barre syndrome, muscular dystrophias, cervical spine injury) or due to chest wall dysfunction (like kyphoscoliosis, extreme obesity, ankylosing spondylitis).

Often patients with chronic respiratory failure present a cor pulmonale or a polycythemia.

### 3-2: Exclusion criteria

Patients meeting one of the following criteria were excluded from enrolment into the study:
1. Legal lower age limitations (country specific).
2. Life expectancy less than 3 months.
3. Known pelvic venous obstruction.
4. Any major orthopedic or general surgery in the 3 months prior to study start.
5. Clinical signs or symptoms of DVT or PE within the last 12 months or known postphlebitic syndrome.
6. Known sensitivity to iodine or contrast dyes, and any contraindications to the performance of venography.
7. Any treatment or procedure with other antithrombotic agents within 2 weeks prior to randomization or planned during the course of the study treatment period that could affect the incidence of VTE, such as:
   - Parenteral anticoagulants (UFH, LMWH [eg, enoxaparin, dalteparin, nadroparin], fondaparinux, bivalirudin, hirudin),
   - Oral anticoagulants (vitamin K antagonists),
   - GPIIb/IIIa antagonists: abciximab, eptifibatide, tirofiban,
   - Thrombolytic agents,
   - Dextrans,
   - Intermittent pneumatic compression of the legs (IPC).
8. Subject unlikely to comply with protocol, eg, uncooperative attitude, inability to return for follow up visits, inability to receive daily injection (preferably by a Health Care Professional) after hospital discharge, and unlikelihood of completing the study.
9. Treatment with any investigational product or investigational device in the last 30 days or 5 half lives (whichever is longer) prior to randomization.
10. Any previous exposure to AVE5026 (eg, participation in any previous AVE5026 clinical trial).
11. Active major bleeding.
12. Thrombocytopenia associated with a positive *in vitro* test for antiplatelet antibody in the presence of enoxaparin sodium.
13. Known hypersensitivity to enoxaparin sodium (eg, pruritus, urticaria, anaphylactoid reactions).
14. Known hypersensitivity to heparin or pork products.
15. Conditions with increased risk of hemorrhage, such as bacterial endocarditis, congenital or acquired bleeding disorders, active ulcerative and angiodysplastic gastrointestinal disease, hemorrhagic stroke, or shortly after brain, spinal, or ophthalmological surgery.
16. End stage renal disease (estimated creatinine clearance <10 mL/min) or patient on dialysis.
17. Pregnant or breastfeeding women.
18. Women of childbearing potential not protected by highly effective contraceptive method of birth control as defined for contraception in the Informed Consent Form for the duration of the study and/or who are unwilling or unable to be tested for pregnancy.

### 4) Treatments

Sanofi-aventis supplied and manufactured the blinded treatments for this study. According to their randomized assignments, patients received either AVE5026 or enoxaparin. Both treatments were presented as a ready-to-use 0.5 mL prefilled syringe, identical in appearance, and containing the same volume of a sterile, isotonic solution with sodium chloride 0.9 % and water for injection.

Enoxaparin treatment was started pre-operatively in accordance with the local enoxaparin labeling. Semuloparin treatment was started post-operatively (8 ±1 hours after the end of the surgery). Due to the different time of administration, pre-filled syringes containing placebo were used. The matching placebo syringe was strictly identical in appearance, containing the same volume but without active component.

AVE5026 or enoxaparin were administered subcutaneously. The entire volume of the pre-filled syringe was injected.

Investigational Product (IP) was administered in a blinded manner once daily during 7-10 days after surgery.

The day of surgery was defined as Day 1. The first pre-operative injection (enoxaparin for patients allocated to comparator group and placebo for patients allocated to AVE5026 group) was administered according to the local enoxaparin labeling but in any case prior to surgery. Then, the first post-operative injection (AVE5026 or placebo) was administered 8 ±1 hours after incision closure, provided that hemostasis had been established. The second post-operative injection (enoxaparin or placebo) was administered 12 ±1 hours after incision closure. When surgery was finalized after 12:00, this IP injection was performed on Day 2. Then, in any case, patients received on the following days (from Day 2 and for 7 to 10 days) one daily IP injections (enoxaparin or AVE5026).

### 5) Assessment of safety

The safety analysis period was defined as the period from the first IP injection up to the last IP injection plus 3 calendar days (called «on-treatment» period). The safety population included all randomized patients exposed to the study treatment, regardless of the number of injections administered.

Safety parameters included, amongst other parameters, bleeding events up to 3 calendar days after last IP injection. All bleedings were adjudicated by a Central Independent Adjudication Committee.

Clinically relevant bleedings and major bleedings were as defined previously. Any reported bleeding not meeting the criteria for major bleeding or clinically relevant non-major bleeding was defined as non-clinically relevant bleeding.

### 6) Results

For clinically relevant non-major bleedings and for major bleedings, event rates per treatment group were calculated, as well as exact 95% CI on the odds ratio, using the mid-p method.

Table 1 describes the incidence of any clinically relevant bleeding events in the safety population of the SAVE-ABDO study, while table 2 describes the incidence of major bleedings in this population.

**Table 1: Number of patients with any treatment-emergent clinically relevant bleedings in the safety population**

| | Semuloparin (N = 2175) | Enoxaparin (N = 2177) |
|---|---|---|
| Any clinically relevant bleeding: | | |
| n (%) | 90 (4.1 %) | 125 (5.7%) |
| (95% mid-p Cl) | (3.4 to 5.0) | (4.8 to 6.8) |
| | | |
| Comparison versus enoxaparin: | | 0.71 (0.54 to 0.93) |
| OR (95% mid-p Cl) | | |

| | | |
|---|---|---|
| N: number of patients in the safety population n: number of patients with any clinically relevant bleeding | | |

These results demonstrate that semuloparin exhibits a statistically significantly better safety profile than enoxaparin in terms of incidence of clinically relevant bleedings, when administered for the prevention of VTE and death to patients who have undergone major abdominal surgery.

**Table 2: Number of patients with treatment-emergent major bleedings in the safety population**

| | Semuloparin (N = 2175) | Enoxaparin (N = 2177) |
|---|---|---|
| Any major bleeding: | | |
| n (%) | 63 (2.9%) | 98 (4.5%) |
| (95% mid-p Cl) | (2.3 to 3.7) | (3.7 to 5.4) |
| | | |
| Comparison versus enoxaparin: | | 0.63 (0.46 to 0.87) |
| OR (95% mid-p Cl) | | |

| | | |
|---|---|---|
| N: number of patients in the safety population n: number of patients with any clinically relevant bleeding | | |

These results demonstrate that semuloparin exhibits a statistically significantly better safety profile than enoxaparin in terms of incidence of major bleedings, when administered for the prevention of VTE and death to patients who have undergone major abdominal surgery.

## Claims

1. Semuloparin or a pharmaceutically acceptable salt thereof for use as an antithrombotic treatment in patients undergoing major abdominal surgery, wherein said use involves an improved safety in terms of clinically relevant bleedings compared to a standard antithrombotic treatment, and wherein said improved safety is clinically proven by a phase III clinical trial.

2. Semuloparin or a pharmaceutically acceptable salt thereof for the use according to claim 1, wherein said use involves an improved safety in terms of major bleedings.

3. Semuloparin or a pharmaceutically acceptable salt thereof for the use according to any claim 1 or claim 2, wherein said standard antithrombotic treatment is a treatment with enoxaparin started pre-operatively.

4. Semuloparin or a pharmaceutically acceptable salt thereof for the use according to any of claim 1 to 3, wherein the administration of semuloparin is started post-operatively.

5. Semuloparin or a pharmaceutically acceptable salt thereof for the use according to any of claim 1 to 4, wherein semuloparin is administered for 7 to 10 days.

6. Semuloparin or a pharmaceutically acceptable salt thereof for the use according to any of claims 1 to 5, wherein semuloparin is administered at a 20 mg daily dose to patients with normal renal function or to patients with mild or moderate renal impairment.

7. Semuloparin or a pharmaceutically acceptable salt thereof for the use according to any of claims 1 to 5, wherein semuloparin is administered at a 10 mg daily dose in patients with severe renal impairment.

8. Semuloparin or a pharmaceutically acceptable salt thereof for the use according to any of claims 1 to 7, wherein semuloparin is administered once daily, starting post-operatively.

9. Semuloparin or a pharmaceutically acceptable salt thereof for the use according to any of claims 1 to 8, wherein said antithrombotic treatment is a prophylactic treatment for venous thromboembolism.

10. An article of manufacture comprising:
- a packaging material,
- a compound chosen from semuloparin or a pharmaceutically acceptable salt thereof, and
- a label or package insert contained within said packaging material indicating that said compound is safe as an antithrombotic treatment in patients undergoing major abdominal surgery.
